# EUROPÄISCHE PATENTSCHRIFT

(11) **EP 0 695 295 B1**
(45) Veröffentlichungstag und Bekanntmachung des Hinweises auf die Patenterteilung: **06.03.2002**
(21) Anmeldenummer: 94913588.3
(22) Anmeldetag: 13.04.1994
(51) Int. Cl.: C07D 239/60, C07D 251/30, C07D 409/12, C07D 401/12, C07D 405/12, A01N 43/54, A01N 43/66, C07C 69/734

(54) **3-(HET)ARYL-CARBONSÄUREDERIVATE, VERFAHREN UND ZWISCHENPRODUKTE ZU IHRER HERSTELLUNG**
3-(HETERO)ARYL CARBOXYLIC ACID DERIVATIVES, METHODS OF PREPARING THEM AND INTERMEDIATES USED IN THEIR PREPARATION
DERIVES DE L'ACIDE 3-(HET)ARYLCARBOXYLIQUE, PROCEDE ET PRODUITS INTERMEDIAIRES POUR LEUR FABRICATION

(30) Priorität: 23.04.1993 DE 4313412
(43) Veröffentlichungstag der Anmeldung: 07.02.1996
(73) Patentinhaber: BASF AKTIENGESELLSCHAFT, 67056 Ludwigshafen (DE)
(72) Erfinder: BAUMANN, Ernst, D-67373 Dudenhofen (DE); RHEINHEIMER, Joachim, D-67063 Ludwigshafen (DE); VOGELBACHER, Uwe, Josef, D-67071 Ludwigshafen (DE); BRATZ, Matthias, D-67346 Speyer (DE); THEOBALD, Hans, D-67117 Limburgerhof (DE); GERBER, Matthias, D-67117 Limburgerhof (DE); WESTPHALEN, Karl-Otto, D-67346 Speyer (DE); WALTER, Helmut, D-67283 Obrigheim (DE); RADEMACHER, Wilhelm, D-67117 Limburgerhof (DE)
(86) Internationale Anmeldenummer: EP9401141
(87) Internationale Veröffentlichungsnummer: WO9425442

(56) Entgegenhaltungen:
- EP-A- 0 409 368
- EP-A- 0 481 512
- EP-A- 0 517 215
- BULLETIN OF THE CHEMICAL SOCIETY OF JAPAN Bd. 49, Nr. 1 , Januar 1976 , TOKYO JP Seiten 341 - 342 VO VAN CHUNG ET AL 'Photochemical reaction of ethyl 3-methyl-3-phenylglycidate in methanol and ether solvents'
- AGRICULTURAL AND BIOLOGICAL CHEMISTRY Bd. 40, Nr. 5 , Mai 1976 , TOKYO JP Seiten 993 - 1000 KATSURA KOGURE ET AL 'On the stereochemistry of ring opening of beta-aryl-beta methylglycidic ester by acids'
- CHEMICAL ABSTRACTS, vol. 85, no. 5, 2. August 1976, Columbus, Ohio, US; abstract no. 32649q, Seite 364 ; & JP,A,7 604 135 (HASEGAWA CO LTD) 14. Januar 1976
- CHEMICAL ABSTRACTS, vol. 119, no. 13, 27. September 1993, Columbus, Ohio, US; abstract no. 139254e, Seite 883 ; in der Anmeldung erwähnt & JP,A,04 356 470 (UBE INDUSTRIES LTD) 10. Dezember 1992

## Beschreibung

3-(Het)aryl-Carbonsäurederivate der allgemeinen Formel I 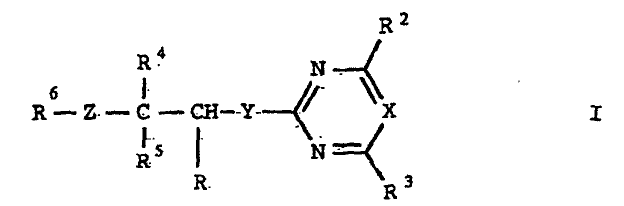 in der R eine Formylgruppe, eine Gruppe CO₂H oder einen zu COOH hydrolysierbaren Rest bedeutet und die übrigen Substituenten folgende Bedeutung haben:
- R²: C₁-C₄-Alkyl, C₁-C₄-Alkoxy;
- X: CR¹⁴, wobei R¹⁴ Wasserstoff bedeute;
- R³: C₁-C₄-Alkyl, C₁-C₄-Alkoxy;
- R⁴: Phenyl, das durch einen oder mehrere, insbesondere ein bis drei der folgenden Reste substituiert sein kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl oder C₁-C₄-Alkoxycarbonyl;
ein fünf- oder sechsgliedriger Heteroaromat, enthaltend ein bis drei Stickstoffatome und/oder ein Schwefel oder Sauerstoffatom, welcher einen oder mehrere der folgenden Reste tragen kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, C₁-C₄-Dialkylamino, C₁-C₄-Alkylcarbonyl, C₁-C₄-Alkoxycarbonyl oder Phenyl;
- R⁵: Wasserstoff, C₁-C₄-Alkyl, C₃-C₈-Cycloalkyl, C₁-C₄-Halogenalkyl oder Phenyl;
- R⁶: C₁-C₈-Alkyl, wobei diese Reste jeweils ein- oder mehrfach substituiert sein können durch: Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
- Y: Sauerstoff;
- Z: Sauerstoff;
mit der Maßgabe, daß R⁶ nicht unsubstituiertes C₁-C₄-Alkyl bedeutet, wenn R⁴ unsubstituiertes Phenyl und gleichzeitig R⁵ Methyl oder Wasserstoff bedeuten.

Im Stand der Technik, z.B. EP-A 347 811, EP-A 400 741, EP-A 409 368, EP-A 481 512, EP-A 517 215, Chemical Abstracts, 119, Nr. 139 254e (1993), und der älteren deutschen Anmeldung P 41 42 570 (EP-A-548 710) werden ähnliche Carbonsäurederivate, unter anderem auch 3-Alkoxyderivate beschrieben, jedoch keine, die einen Het(aryl)-Rest in 3-Stellung tragen.

Darüber hinaus werden im Stand der Technik Zwischenprodukte beschrieben, die zur Herstellung der 3-(Het)aryl-carbonsäurederivate geeignet sind.

So ist aus dem BULLETIN OF THE CHEMICAL SOCIETY OF JAPAN, Bd. 49, Nr. 1, Januar 1976, Seiten 341-342 die photochemische Herstellung von 3-Methoxy-3-phenyl-2-hydroxybuttersäureethylester bekannt. In diesem Dokument wird der Einfluß der Lösungsmittel auf die Reaktionsprodukte untersucht.

Die AGRICULTURAL AND BIOLOGICAL CHEMISTRY, Bd. 40, Nr. 5, Mai 1976, Seiten 993-1000 beschreibt die Stereochemie der Ringöffnung von β-Aryl-β-methylglycidylestern mit Säuren, wobei auch 3-Methoxy-3-(4-isobutylphenyl)-2-hydroxybuttersäuremethylester entstehen.

Aus CHEMICAL ABSTRACTS, vol. 85, no. 5, 2. August 1976, abstract no. 32649q, Seite 364; & JP,A,7 604 135 ist die Herstellung von 3-Phenylglycerinsäurederivaten aus 3-Phenylglycidylsäurederivaten bekannt.

Da die herbizide und/oder bioregulatorische Wirkung und Selektivität der bekannten Verbindungen nicht immer befriedigend ist, lag der Erfindung die Aufgabe zugrunde, Verbindungen mit besserer Selektivität und/oder besserer biologischer Wirkung bereitzustellen.

Es wurde nun gefunden, daß die eingangs definierten 3-(Het)aryl-Carbonsäurederivate ausgezeichnete herbizide und pflanzenwachstumsregulierende Eigenschaften haben. Darüber hinaus weisen die Verbindungen I eine gute pharmakologische Wirksamkeit, insbesondere auf dem Herz-/Kreislaufgebiet auf.

Die Herstellung der erfindungsgemäßen Verbindungen geht aus von den Epoxiden IV, die man in allgemein bekannter Weise, z.B. wie in J. March, Advanced Organic Chemistry, 2nd ed., 1983, S. 862 und S. 750 beschrieben, aus den Aldehyden bzw. Ketonen II oder den Olefinen III erhält:

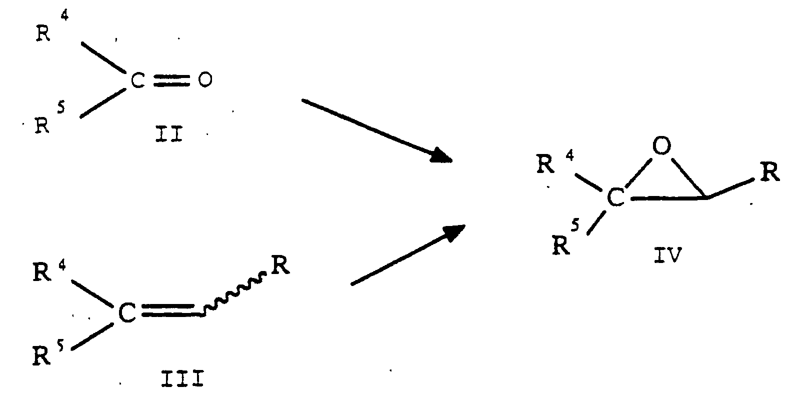

3-(Het)aryl-Carbonsaurederivate der allgemeinen Formel VI können hergestellt werden, indem man die Epoxide der allgemeinen Formel IV (z.B. mit R = ROOR¹⁰) mit Alkoholen oder Thiolen der allgemeinen Formel V, in der R⁶ und Z die in Anspruch 1 genannte Bedeutung haben, zur Reaktion bringt.

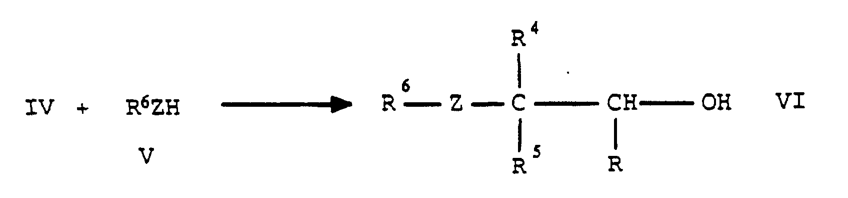

Dazu werden Verbindungen der allgemeinen Formel IV mit einem Überschuß der Verbindungen der Formel V, z.B. 1,2-7, bevorzugt 2-5 Molaquivalenten, auf eine Temperatur von 50 - 200°C, bevorzugt 80 - 150°C, erhitzt.

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei starke organische und anorganische Säuren sowie Lewissäuren in Frage. Beispiele hierfür sind unter anderem Schwefelsäure, Salzsäure, Trifluoressigsäure, Bortrifluorid-Etherat und Titan (IV)-Alkoholate.

Die erfindungsgemäßen Verbindungen, in denen Y Sauerstoff bedeutet und die restlichen Substituenten die unter der allgemeinen Formel I angegebenen Bedeutung haben, können beispielsweise derart hergestellt werden, daß man die 3-(Het)aryl-Carbonsäurederivate der allgemeinen Formel VI, in denen die Substituenten die angegebene Bedeutung haben, mit Verbindungen der allgemeinen Formel VII, 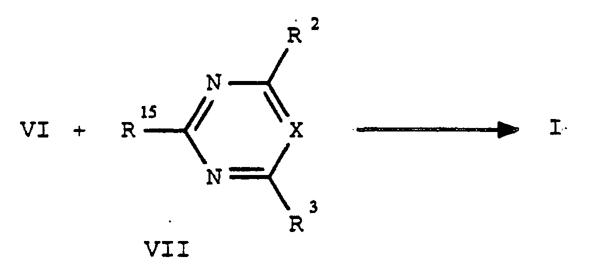 in der R¹⁵ Halogen oder R¹⁶-SO₂- bedeutet, wobei R¹⁶ C₁-C₄-Alkyl, C₁-C₄-Halogenalkyl oder Phenyl sein kann, zur Reaktion bringt. Die Reaktion findet bevorzugt in einem der oben genannten inerten Verdünnungsmittel unter Zusatz einer geeigneten Base, d.h. einer Base, die eine Deprotonierung des Zwischenproduktes VI bewirkt, in einem Temperaturbereich von Raumtemperatur bis zum Siedepunkt des Lösungsmittels statt.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, eine metallorganische Verbindung wie Butyllithium oder ein Alkaliamid wie Lithiumdiisopropylamid dienen.

Die erfindungsgemäßen Verbindungen, in denen Y Schwefel bedeutet und die restlichen Substituenten die unter der allgemeinen Formel I angegebene Bedeutung haben, können beispielsweise derart hergestellt werden, daß man 3-(Het)aryl-Carbonsäurederivate der allgemeinen Formel VIII, die in bekannter Weise aus Verbindungen der allgemeinen Formel VI erhältlich sind und in denen die Substituenten die oben angegebene Bedeutung haben, mit Verbindungen der allgemeinen Formel IX, in der R², R³ und X die unter der allgemeinen Formel I angegebene Bedeutung haben, zur Reaktion bringt.

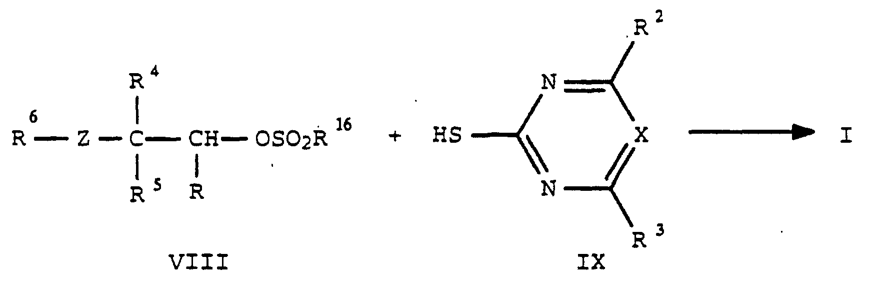

Die Reaktion findet bevorzugt in einem der oben genannten inerten Verdünnungsmittel unter Zusatz einer geeigneten Base, d.h. eine Base, die eine Deprotonierung des Zwischenproduktes IX bewirkt, in einem Temperaturbereich von Raumtemperatur bis zum Siedepunkt des Lösungsmittels statt.

Als Base können neben den oben genannten auch organische Basen wie tertiäre Amine, z.B. Triethylamin, Pyridin, Imidazpl oder Diazabicycloundecan dienen.

Verbindungen der Formel I können auch dadurch hergestellt werden, daß man von den entsprechenden Carbonsäuren, d. h. Verbindungen der Formel I, in denen R¹ Hydroxyl bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR¹⁰ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsauren ausgeht, d. h. von Verbindungen der Formel I, in denen R für eine Gruppe COR¹ und R¹ für OM stehen, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und wird vorteilhaft unter Zugabe einer Base, wobei die oben genannten in Betracht kommen, vorgenommen.

Der Rest R in Formel I ist breit variabel. Beispielsweise steht R für eine Gruppe 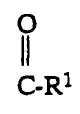 in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Halogenalkoxyl insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) R¹ ferner ein Rest 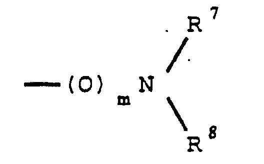 in dem m für 0 oder 1 steht und R⁷ und R⁸, die gleich oder unterschiedlich sein können, die folgende Bedeutung haben:
   Wasserstoff
   C₁-C₈-Alkyl, insbesondere C₁-C₄-Alkyl wie oben genannt;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl,-1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl
   C₃-C₈-Cycloalkyl, wie Cyclopropyl, Cyclobutyl, Cyclopentyl, Cyclohexyl und Cycloheptyl, Cyclooctyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
      C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
      C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
      C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
      C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
      Phenyl, gegebenenfalls ein- oder mehrfach, z.B. ein- bis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
      Di-C₁-C₄-Alkylamino wie insbesondere Dimethylamino, Dipropylamino, N-Propyl-N-methylamino, N-Propyl-N-ethylamino, biisopropylamino, N-Isopropyl-N-methylamino, N-Isopropyl-N-ethylamino, N-Isopropyl-N-propylamino;
      R⁷ und R⁸ ferner Phenyl, das durch einen oder mehrere, z.B. ein bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wie insbesondere oben genannt;
   oder R⁷ und R⁸ bilden gemeinsam eine zu einem Ring geschlossene, optionell substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-;
e) R¹ ferner eine Gruppe 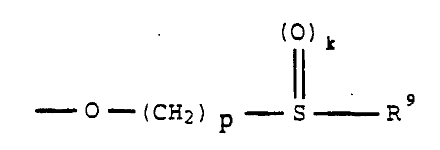 in der k die Werte 0, 1 und 2, p die Werte 1, 2, 3 und 4 annehmen und R⁹ für
   C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere oben genannt.
f) R¹ ferner ein Rest OR¹⁰, worin R¹⁰ bedeutet:
   Wasserstoff, das Kation eines Alkalimetalls wie Lithium, Natrium, Kalium oder das Kation eines Erdalkalimetalls wie Calcium, Magnesium und Barium oder ein umweltverträgliches organisches Ammoniumion wie tertiäres C₁-C₄-Alkylammonium oder das Ammoniumion;
   C₃-C₈-Cycloalkyl wie vorstehend genannt, welches ein bis drei C₁-C₄-Alkylgruppen tragen kann;
   C₁-C₈-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/oder einen der folgenden Reste tragen kann:
      C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
      eine C₁-C₈-Alkylgruppe wie vorstehend genannt, welch ein bis fünf Halogenatome, insbesonder Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
         Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-l-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
         eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
         eine C₃-C₆-Alkenyl- oder eine C₃-C₆-Alkinylgruppe, wobei diese Gruppen ihrerseits ein bis fünf Halogenatome tragen können;
         R¹⁰ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
         ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
         R¹⁰ ferner ein Gruppe 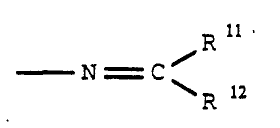
   worin R¹¹ und R¹², die gleich oder verschieden sein können, bedeuten:
   C₁-C₈-Alkyl, C₃-C₆-Alkenyl, C₃-C₆-Alkinyl, C₃-C₈-Cycloalkyl. wobei diese Reste einen C₁-C₄-Alkoxy, C₁-C₄-Alkylthio und/oder einen gegebenenfalls substituierten Phenylrest, wie insbesondere vorstehend genannt, tragen können;
   Phenyl, das durch einen oder mehrere, z.B. einen bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben genannten entsprechen;
   oder R¹¹ und R¹² bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁷ und R⁸ genannt.
g) R¹ ferner ein Rest 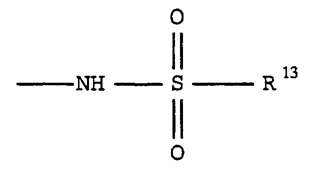 worin R¹³ bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.

Im Hinblick auf die biologische Wirkung sind 3-(Het)aryl-Carbonsäurederivate der allgemeinen Formel I bevorzugt, in denen die Substituenten folgende Bedeutung haben:
- R²: die bei R¹ im einzelnen genannten C₁-C₄-Alkyl-, C₁-C₄-Alkoxy-, insbesondere Methyl, Methoxy, Ethoxy, besonders bevorzugt Methoxy;
- X: CR¹⁴, worin
- R¹⁴: Wasserstoff bedeutet;
- R³: die bei R¹ genannten C₁-C₄-Alkyl-, C₁-C₄-Alkoxy-, insbesondere Methyl, Methoxy, Ethoxy, besonders bevorzugt steht R³ für Methoxy;
- R⁴: ein 5- oder 6-gliedriges Heteroaryl wie Furyl, Thienyl, Pyrryl, Pyrazolyl, Imidazolyl, Triazolyl, Isoxazolyl, Oxazolyl, Isothiazolyl, Thiazolyl, Thiadiazolyl, Pyridyl, Pyrimidinyl, Pyrazinyl, Pyridazinyl, Triazinyl, beispielsweise 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazoylyl, Thia-2,4-diazolyl, Thia-3,4-diazolyl und Triazolyl, wobei die Heteroaromaten ein bis fünf Halogenatome wie vorstehend genannt, insbesondere Fluor und Chlor und/oder einen bis drei der folgenden Reste tragen können:
C₁-C₄-Alkyl, C₁-C₄-Alkoxy, C₁-C₄-Alkylthio, Cyano, Nitro, C₁-C₈-Alkylcarbonyl, C₁-C₈-Alkoxycarbonyl, Phenyl, Phenoxy, Phenylcarbonyl wie im allgemeinen und besonderen oben genannt;
R⁴ ferner Phenyl, das durch einen oder mehrere, z.B. einen bis drei der folgenden Reste substituiert sein können: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl, insbesondere wie bei R⁷ und R⁸ genannt, sowie beispielsweise 3-Hydroxyphenyl, 4-Dimethylaminophenyl, 2-Mercaptophenyl, 3-Methoxycarbonylphenyl, 4-Acetylphenyl;
- R⁵: Wasserstoff, C₁-C₄-Alkyl, C₃-C₈-Cycloalkyl, C₁-C₄-Halogenalkyl oder Phenyl;
- R⁶: C₁-C₈-Alkyl, wie insbesondere oben genannt, wobei diese Reste jeweils ein-oder mehrfach substituiert sein können durch: Phenyl, ein oder mehrfach durch Halogen, Nitro, Cyano, C₁-C₄-Alkyl, C₁-C₄-Halogenalkyl, C₁-C₄-Alkoxy, C₁-C₄-Alkylthio oder C₁-C₄-Halogenalkoxy substituiertes Phenyl oder Phenoxy;
- Y: Sauerstoff;
- Z: Sauerstoff,
mit der Maßgabe, daß R⁶ nicht unsubstituiertes C₁-C₄-Alkyl bedeutet, wenn R⁴ unsubstituiertes Phenyl, Z Sauerstoff und gleichzeitig R⁵ Methyl oder Wasserstoff bedeuten.

Besonders bevorzugt sind Verbindungen der Formel I, in denen R² und R³ Methoxy und X CH bedeuten. Beispiele für bevorzugte Verbindungen sind in der nachfolgenden Tabelle aufgeführt. Die darin und in der Tabelle 1 und 2 für R⁴ genannten Definitionen sind ebenfalls als bevorzugt anzusehen, unabhängig von den mit R⁴ kombinierten Restedefinitionen.

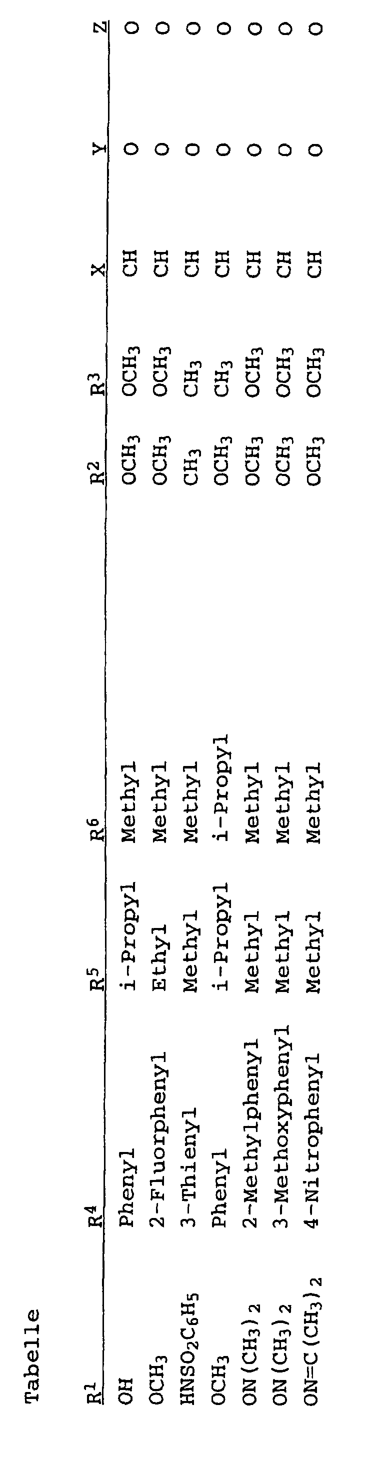

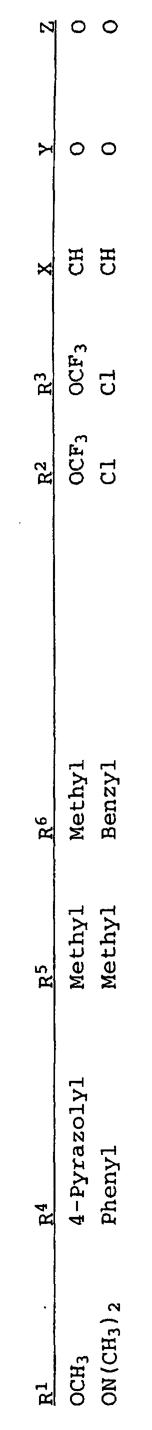

Die Verbindungen I bzw. die sie enthaltenden herbiziden Mittel sowie deren umweltverträgliche Salze von Alkalimetallen und Erdalkalimetallen können in Kulturen wie Weizen, Reis und Mais, Soja und Baumwolle, Unkräuter und Schadgräser sehr gut bekämpfen, ohne die Kulturpflanzen zu schädigen, ein Effekt, der vor allem auch bei niedrigen Aufwandmengen auftritt.

Sie können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen u.a. Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 bis 100 %, vorzugsweise 95 bis 100 % (nach NMR-Spektrum) eingesetzt.

Beispiele für Formulierungen sind:
I. 20 Gewichtsteile der Verbindung Nr. 2.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 2.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. 2.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 2.1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
V. 3 Gewichtsteile des Wirkstoffs Nr. 2.1 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 2.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5 kg/ha, vorzugsweise 0,01 bis 2 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium, herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., P, isum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera, Zea mays.

Die Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert.
   Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blatt-wachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensität der Bewässerung reduzieren und damit eine kostengünstigere Bewirtschaftung durchführen, weil u.a.
   - die Öffnungsweite der Stomata reduziert wird,
   - eine dickere Epidermis und Cuticula ausgebildet werden,
   - die Durchwurzelung des Bodens verbessert wird und
   - das Mikroklima im Pflanzenbestand durch einen kompakteren Wuchs günstig beeinflußt wird.

Besonders gut eignen sich sich Verbindungen I zur Halmverkürzung von Kulturpflanzen wie Gerste, Raps und Weizen.

Die erfindungsgemäß zu verwendenden Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, bevorzugt 0.01 bis 3 kg/ha, insbesondere 0.01 bis 0.5 kg/ha als ausreichend zu betrachten.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy- bzw. Heteroaryloxy-phenoxypropionsäuren sowie deren Salze, Ester und Amide und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Synthesebeispiele

### Synthese von Verbindungen der allgemeinen Formel VI

### Beispiel 1

### 3-Methoxy-3-(3-methoxyphenyl)-2-hydroxybuttersäuremethylester

19,5 g (88 mmol) 3-(3-Methoxyphenyl)-2,3-epoxybuttersäuremethylester werden in 200 ml absolutem Methanol gelöst und mit 0,1 ml Bortrifluorid-Etherat versetzt. Man rührt 12 Stunden bei Raumtemperatur und destilliert das Lösungsmittel ab. Der Rückstand wird in Essigester aufgenommen, mit Natriumbicarbonat-Lösung und Wasser gewaschen und über Natriumsulfat getrocknet. Nach Abdestillieren des Lösungsmittels verbleiben 21,1 g eines schwach gelben Öls.

Ausbeute: 94 % (Diastereomerengemisch 1:1)

### Beispiel 2

### 3-Benzyloxy-3-phenyl-2-hydroxybuttersäuremethylester

9,6 g (50 mmol) 3-Phenyl-2,3-epoxybuttersäuremethylester werden in 150 ml Benzylalkohol gelöst und mit 0,5 ml konzentrierter Schwefelsäure versetzt. Man rührt 6 Stunden bei 50°C und läßt auf Raumtemperatur abkühlen. Nach Neutralisation mit Natriumbicarbonat-Lösung destilliert man den überschüssigen Benzylalkohol am Hochvakuum ab und reinigt den Rückstand durch Flash-Chromatographie an Kieselgel mit n-Hexan/Essigester 9:1. Nach Abdestillieren des Lösungsmittels verbleiben 6,5 g eines farblosen Öls.

Ausbeute: 43 % (Diastereomerengemisch 3:2)

Analog wurden alle in Tabelle 1 genannten Verbindungen hergestellt.

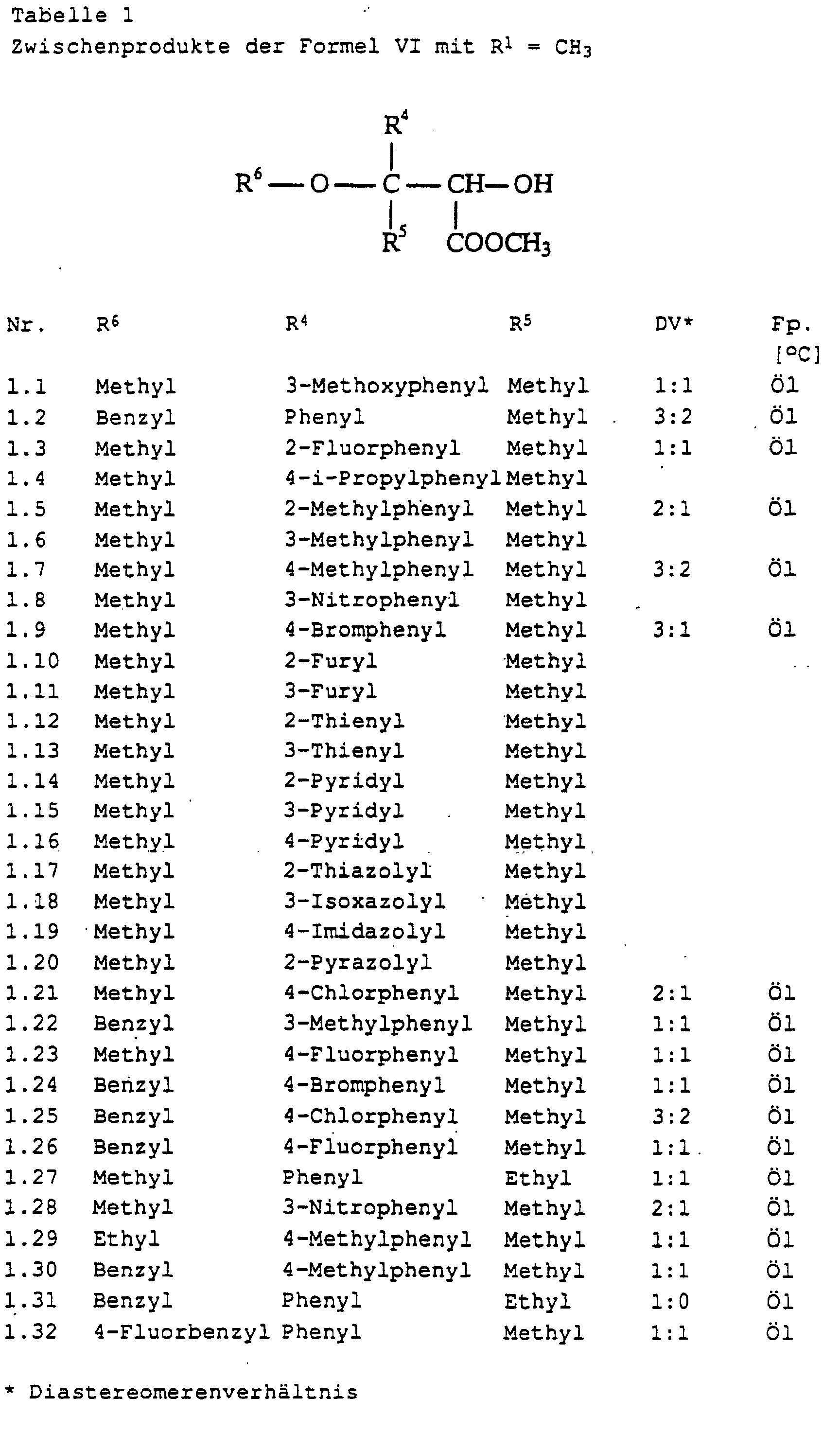

### Synthese von Verbindungen der allgemeinen Formel I:

### Beispiel 3:

### 3-Benzyloxy-3-phenyl-2-(4,6-dimethoxypyrimidin-2-yl)oxybuttersäure-methylester

3 g (10 mmol) 3-Benzyloxy-3-phenyl-2-hydroxybuttersäuremethylester (Verb. 1.1) werden in 40 ml Dimethylformamid gelöst und mit 0,3g (12mmol) Natriumhydrid versetzt. Man rührt 1 Stunde und gibt dann 2,2 g (10 mmol) 4,6-Dimethoxy-2-methylsulfonylpyrimidin zu. Nach 24 Stunden Rühren bei Raumtemperatur wird vorsichtig mit 10 ml Wasser hydrolisiert, mit Essigsäure ein pH-Wert von 5 eingestellt und das Lösungsmittel am Hochvakuum abdestilliert. Der Rückstand wird in 100 ml Essigester aufgenommen, mit Wasser gewaschen, über Natriumsulfat getrocknet und das Lösungsmittel abdestilliert. Der Rückstand wird mit 10 ml Methyl-t-butylether versetzt und der gebildete Niederschlag abgesaugt. Nach dem Trocknen verbleiben 2,4g eines weißen Pulvers.

Ausbeute: 55 % (Diastereomerengemisch 1:1)
Fp.: 115 - 117°C

### Beispiel 4

### 3-Benzyloxy-3-phenyl-2-(4,6-dimethoxypyrimidin-2-yl)oxybuttersäure

1,4 g (3 mmol) 3-Benzyloxy-3-phenyl-2-(4,6-dimethoxypyrimidin-2-yl)-oxybuttersäuremethylester (Bsp. 3) werden in 20 ml Methanol und 20 ml Tetrahydrofuran gelöst und mit 3,7 g 10 % NaOH-Lösung versetzt. Man rührt 6 Stunden bei 60°C und 12 Stunden bei Raumtemperatur, destilliert die Lösungsmittel im Vakuum ab und nimmt den Rückstand in 100 ml Wasser auf. Nun wird mit Essigester zur Entfernung von nicht umgesetztem Ester extrahiert. Anschließend stellt man die Wasserphase mit verdünnter Salzsäure auf pH 1-2 und extrahiert mit Essigester. Nach Trocknen über Magnesiumsulfat und Abdestillieren des Lösungsmittels wird der Rückstand mit wenig Aceton versetzt und der gebildete Niederschlag abgesaugt. Nach dem Trocknen verbleiben 1,2 g eines weißen Pulvers.

Ausbeute: 88 %
Fp.: 165°C (Zersetzung, Diastereomerengemisch 3:2)

### Beispiel 5

### 3-Benzyloxy-3-phenyl-2-[(4,6-dimethoxypyrimidin-2-yl)thio]-buttersäuremethylester

11 g (25 mmol) 3-Benzyloxy-3-phenyl-2-hydroxybuttersäuremethylester (Verb. 1.1) werden in 50 ml Dichlormethan gelöst, 3 g (30 mmol) Triethylamin zugegeben und unter Rühren 3,2 g (28 mmol) Methansulfonsäurechlorid zugetropft. Man rührt 2 Stunden bei Raumtemperatur, wäscht mit Wasser, trocknet über Magnesiumsulfat und engt im Vakuum ein. Der Rückstand wird in DMF aufgenommen und bei 0°C zu einer Suspension von 12,9 g (75 mmol) 4,6-Dimethoxypyrimidin-2-thiol und 8,4 g (100mmol) Natriumhydrogencarbonat in 100 ml DMF getropft. Nach 2 Stunden Rühren bei Raumtemperatur und weiteren 2 Stunden bei 60°C gießt man auf 1 1 Eiswasser und saugt den entstandenen Niederschlag ab. Nach Trocknen verbleiben 3,2 g eines weißen Pulvers.

Ausbeute: 29 % (Diastereomerengemisch 1:1)

Analog den obigen Beispielen wurden die in Tabelle 2 genannten Verbindungen hergestellt.

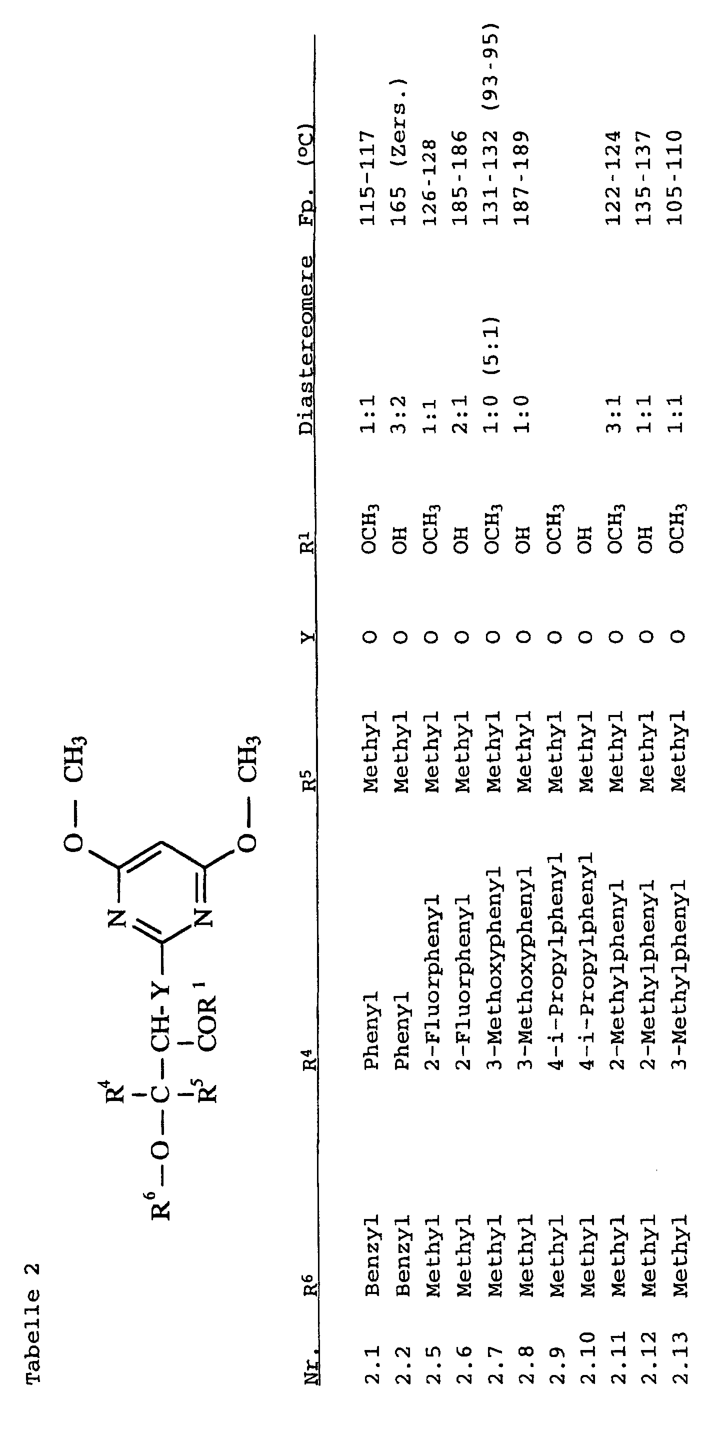

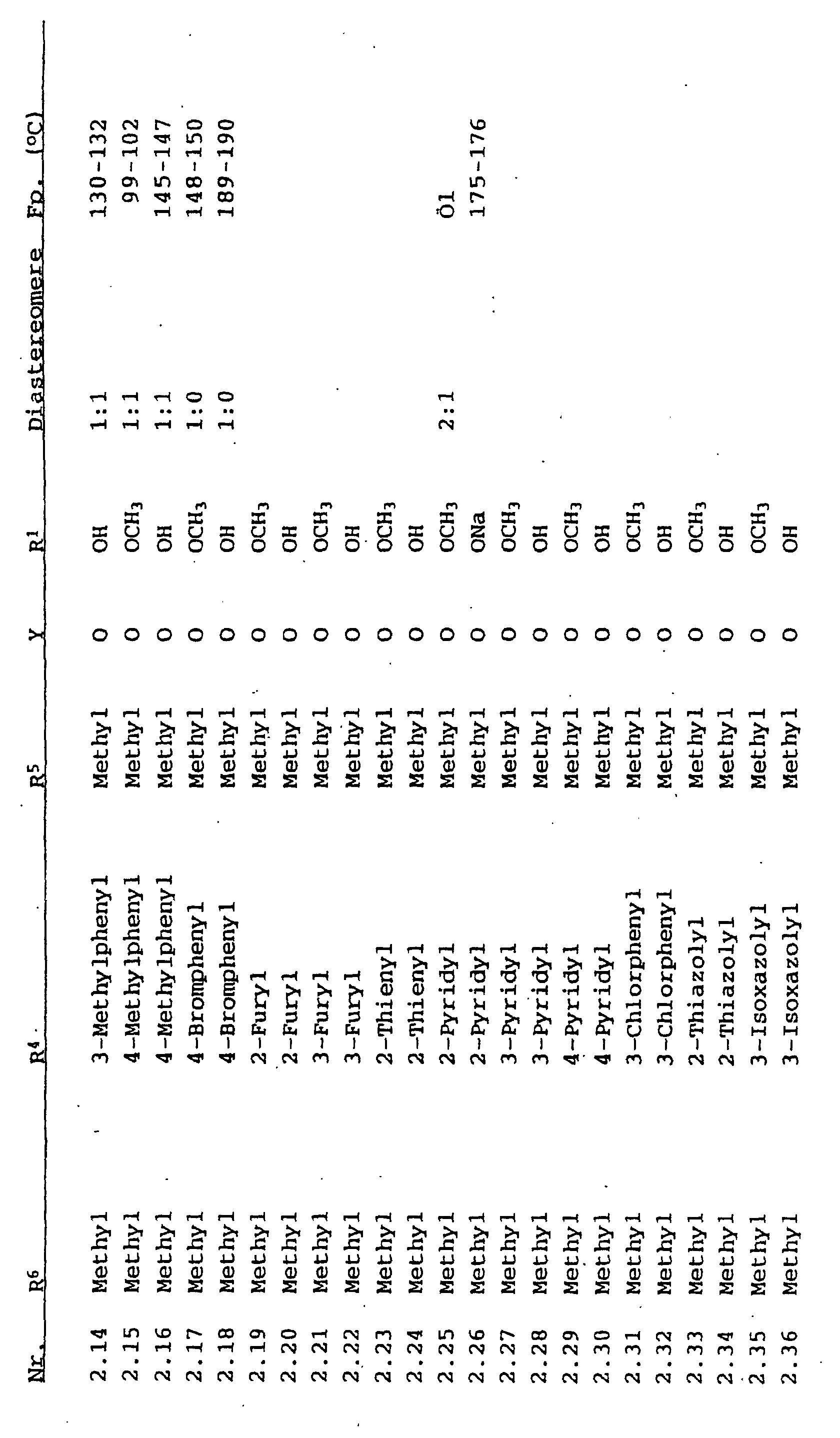

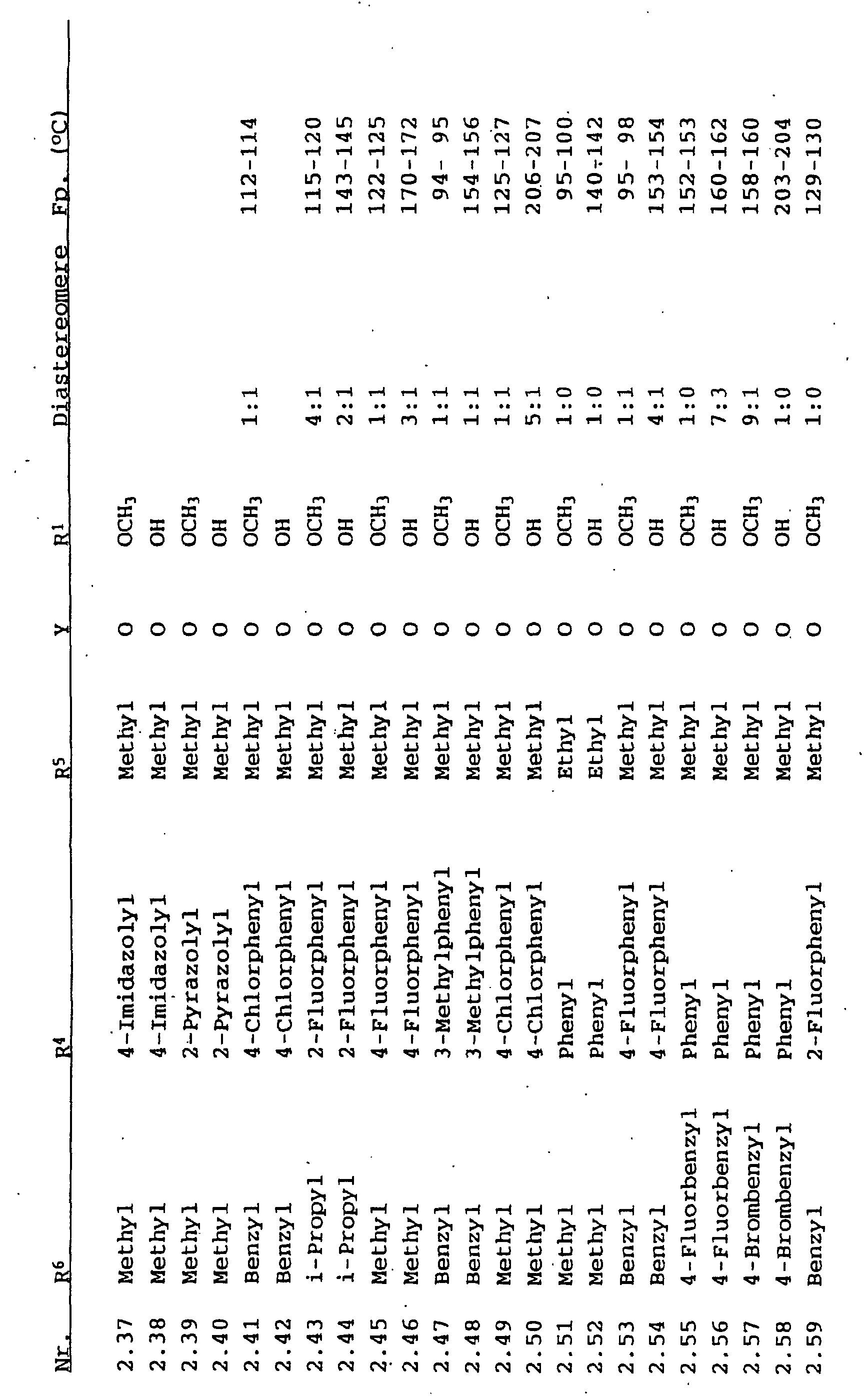

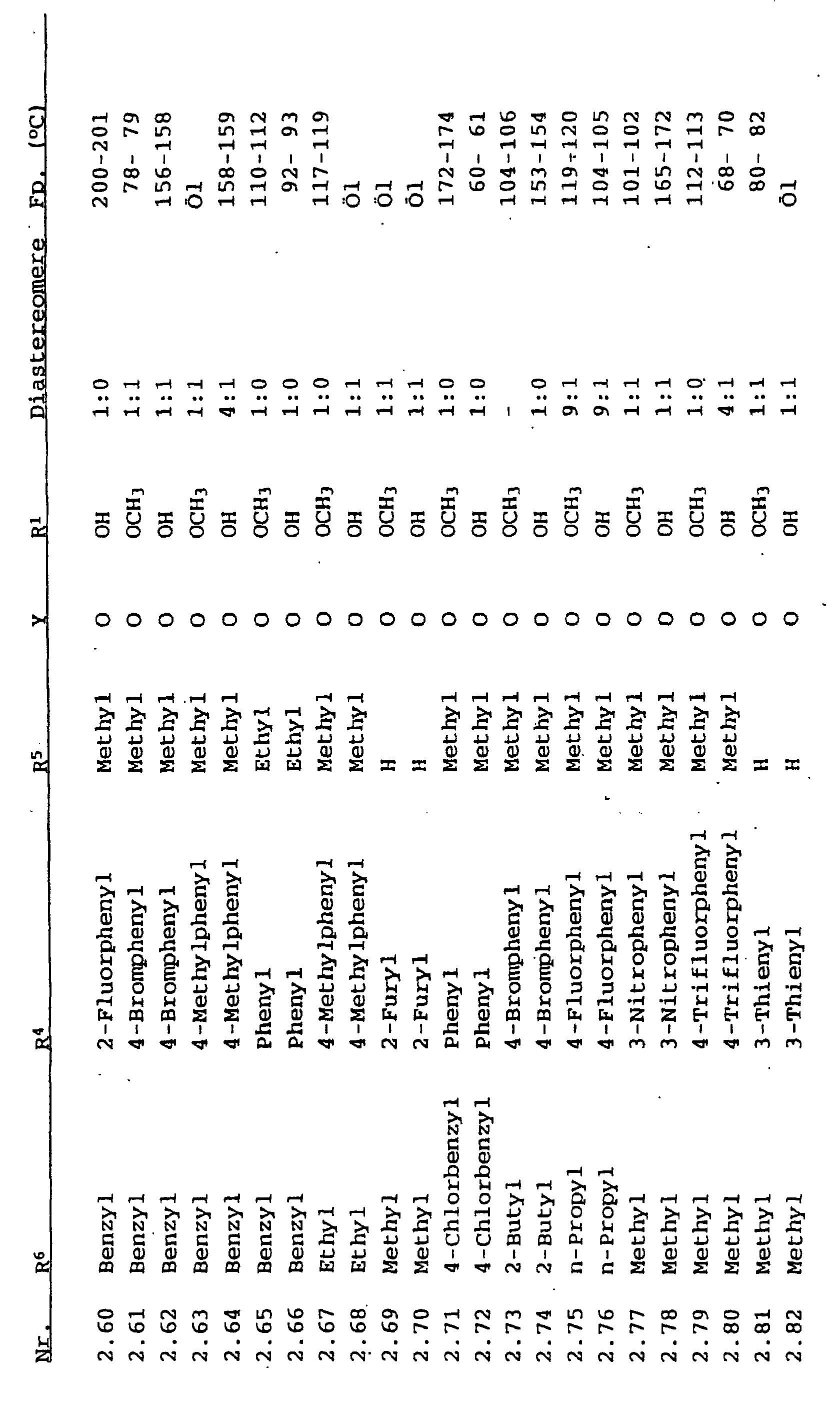

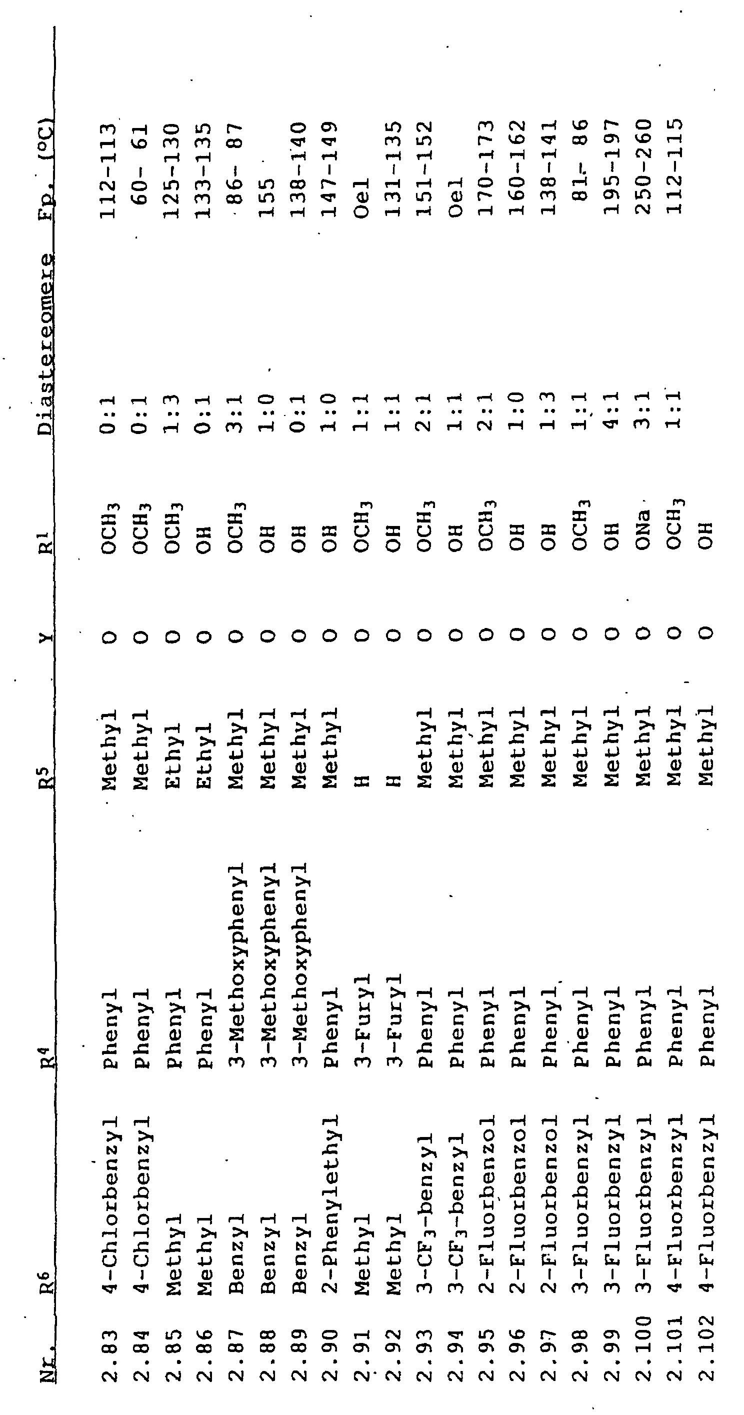

### Anwendungsbeispiele

Die herbizide Wirkung der 3-(Het)aryl-carbonsäurederivate der allgemeinen Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufanwendung werden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung beträgt 0,125 bzw. 0,06 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Abkürzung |
|---|---|---|
| Gossypium hirsutum | Baumwolle | GOSHI |
| Oryza sativa | Reis | ORYSA |
| Triticum aestivum | Sommerweizen | TRZAS |
| Alopecurus myosuroides | Ackerfuchsschwanz | ALOMY |
| Amaranthus retroflexus | Amarant | AMARE |
| Brachiaria platyphylla | - | BRAPP |
| Chenopodium album | Weißer Gänsefuß | CHEAL |
| Sesbania exaltata | Turibaum | SEBEX |
| Setaria faberii | Große Borstenhirse | SETFA |
| Setaria viridis | Grüne Borstenhirse | SETVI |
| Solanum nigrum | Schwarzer Nachtschatten | SOLNI |
| Veronica spp. | Ehrenpreisarten | VERSS |

Die in Tabelle A zusammengestellten Ergebnisse zeigen die überlegene herbizide Wirkung sowie die bessere Selektivität der erfindungsgemäßen Verbindung Nr. 2.2 im Vergleich zu der aus EP-A 409 368 bekannten Vergleichssubstanz A

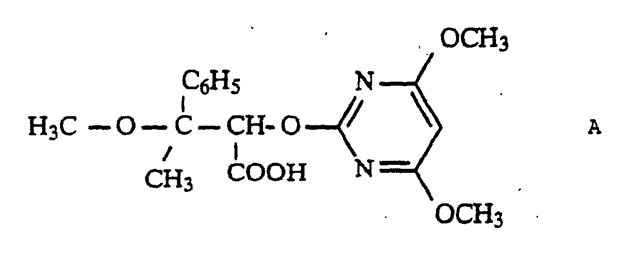

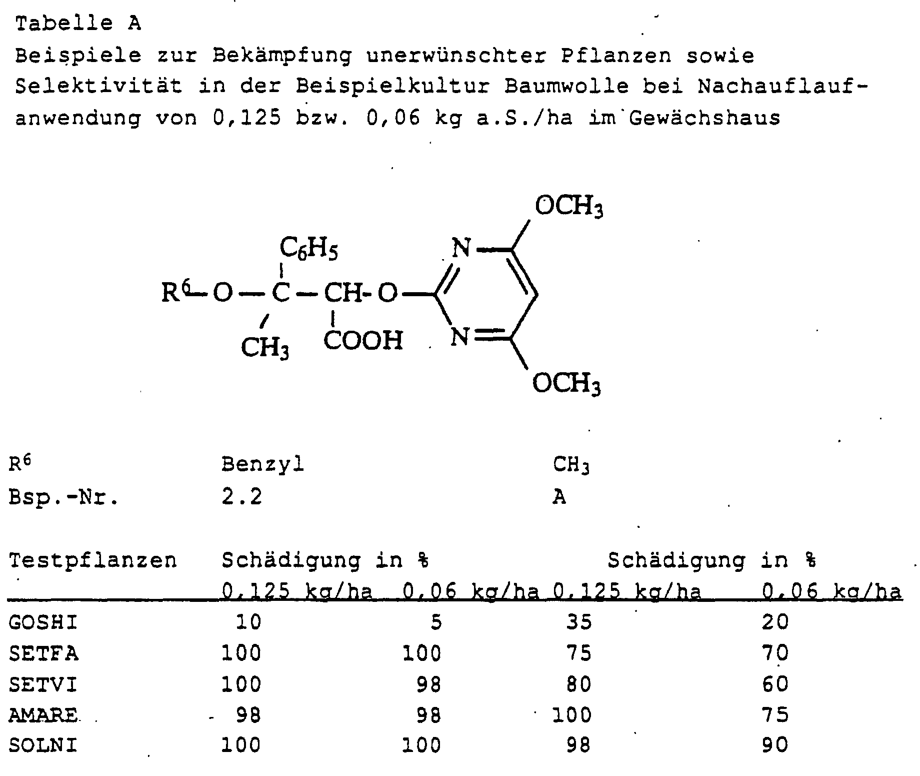

Verbindungen Nr. 2.84, 2.16, 2.52, 2.86 und 2.25 zeigten bei Aufwandmengen von 5 kg/ha bis 0,25 kg/ha gute herbizide Wirkung. Dabei wiesen Verbindungen Nr. 2.84 und 2.16 gleichzeitig eine sehr gute Selektivität in der Beispielkultur Baumwolle auf. Darüber hinaus war Beispiel Nr. 2.16 auch in Reis selektiv. Beispiel 2.52 wurde gut von der Kulturpflanze Sommerweizen toleriert.

## Patentansprüche

1. 3-(Het)aryl-Carbonsäurederivate der allgemeinen Formel I 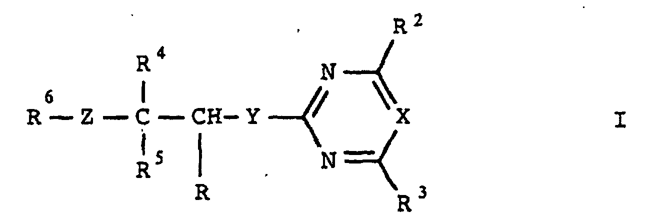 in der R eine Formylgruppe, eine Gruppe CO₂H oder einen zu COOH hydrolysierbaren Rest bedeutet und die übrigen Substituenten folgende Bedeutung haben:
R² C₁-C₄-Alkyl, C₁-C₄-Alkoxy;
X CR¹⁴, wobei R¹⁴ Wasserstoff bedeutet;
R³ C₁-C₄-Alkyl, C₁-C₄-Alkoxy;
R⁴ Phenyl, das durch einen oder mehrere, insbesondere ein bis drei der folgenden Reste substituiert sein kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl oder C₁-C₄-Alkoxycarbonyl;
ein fünf- oder sechsgliedriger Heteroaromat, enthaltend ein bis drei Stickstoffatome und/oder ein Schwefel- oder Sauerstoffatom, welcher einen oder mehrere der folgenden Reste tragen kann: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, C₁-C₄-Dialkylamino, C₁-C₄-Alkylcarbonyl, C₁-C₄-Alkoxycarbonyl oder Phenyl;
R⁵ Wasserstoff, C₁-C₄-Alkyl, C₃-C₈-Cycloalkyl, C₁-C₄-Halogenalkyl oder Phenyl;
R⁶ C₁-C₈-Alkyl, wobei dieser Rest jeweils ein- oder mehrfach substituiert sein kann durch: Phenyl, ein-oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
Y Sauerstoff;
Z Sauerstoff;
mit der Maßgabe, daß R⁶ nicht unsubstituiertes C₁-C₄-Alkyl bedeutet, wenn R⁴ unsubstituiertes Phenyl, Z Sauerstoff und gleichzeitig R⁵ Methyl oder Wasserstoff bedeuten.

2. 3-(Het)aryl-Carbonsäurederivate der allgemeinen Formel I gemäß Anspruch 1, in der R für eine Gruppe 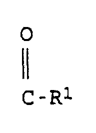 steht, wobei R¹ die folgende Bedeutung hat:
a) Wasserstoff;
b) eine Succinylimidoxygruppe;
c) ein über ein Stickstoffatom verknüpfter 5-gliedriger Heteroaromat, enthaltend zwei bis drei Stickstoffatome, welcher ein bis zwei Halogenatome und/oder ein bis zwei der folgenden Reste tragen kann:
C₁-C₄-Alkyl, C₁-C₄-Halogenalkyl, C₁-C₄-Alkoxy,
C₁-C₄-Halogenalkoxy und/oder C₁-C₄-Alkylthio;
d) ein Rest 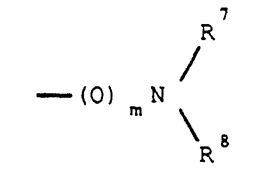 in dem m für 0 oder 1 steht und R⁷ und R⁸, die gleich oder unterschiedlich sein können, die folgende Bedeutung haben:
Wasserstoff;
C₁-C₈-Alkyl, C₃-C₆-Alkenyl, C₃-C₆-Alkinyl, C₃-C₈-Cycloalkyl, wobei diese Reste jeweils ein bis fünf Halogenatome und/oder ein bis zwei der folgenden Gruppen tragen können: C₁-C₄-Alkoxy, C₃-C₆-Alkenyloxy, C₃-C₆-Alkinyloxy, C₁-C₄-Alkylthio, C₃-C₆-Alkenylthio, C₃-C₆-Alkinylthio, C₁-C₄-Halogenalkoxy, C₁-C₄-Alkylcarbonyl, C₃-C₆-Alkenylcarbonyl, C₃-C₆-Alkinylcarbonyl, C₁-C₄-Alkoxycarbonyl, C₃-C₆-Alkenyloxycarbonyl, C₃-C₆-Alkinyloxycarbonyl, Di-C₁-C₄-alkylamino, C₃-C₈-Cycloalkyl, Phenyl, ein oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
R⁷ und R⁸ gemeinsam eine zu einem Ring geschlossene, optionell substituierte C₄-C₇-Alkylenkette oder gemeinsam eine zu einem Ring geschlossene, optionell substituierte C₃-C₆-Alkylenkette mit einem Heteroatom, ausgewählt aus der Gruppe Sauerstoff, Schwefel oder Stickstoff;
e) R¹ ferner eine Gruppe 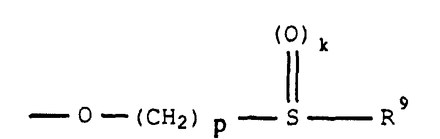 in der R⁹ für C₁-C₄-Alkyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl, C₁-C₄-Halogenalkyl, C₃-C₆-Alkenyl oder C₃-C₆-Alkinyl steht, p die Werte 1, 2, 3 oder 4 und k die Werte 0, 1 oder 2 annehmen;
f) ein Rest OR¹⁰, worin R¹⁰ bedeutet:
i) Wasserstoff, ein Alkalimetallkation, das Äquivalent eines Erdalkalimetallkations, das Ammoniumkation oder ein organisches Ammoniumion;
ii) eine C₃-C₈-Cycloalkylgruppe, welche ein bis drei C₁-C₄-Alkylreste tragen kann;
iii) eine C₁-C₈-Alkylgruppe, welche ein bis fünf Halogenatome und/oder einen der folgenden Reste tragen kann:
C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl,
C₁-C₄-Halogenalkyl, C₁-C₄-Alkoxy, C₁-C₄-Halogenalkoxy und/oder C₁-C₄-Alkylthio;
iv) eine C₁-C₈-Alkylgruppe, welche ein bis fünf Halogenatome tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
v) eine C₂-C₆-Alkylgruppe, welche in der 2-Position einen der folgenden Reste trägt: C₁-C₄-Alkoxyimino, C₃-C₆-Alkenyloxyimino, C₃-C₆-Halogenalkenyloxyimino oder Benzyloxyimino;
vi) eine C₃-C₆-Alkenyl- oder eine C₃-C₆-Alkinylgruppe, wobei diese Gruppen ihrerseits ein bis fünf Halogenatome tragen können;
vii) ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
viii) ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
ix) R¹⁰ ferner eine Gruppe 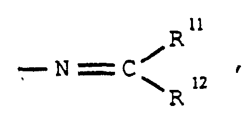 worin R¹¹ und R¹², die gleich oder verschieden sein können, bedeuten:
C₁-C₈-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch einen oder mehrere der folgenden Reste substituiert sein kann;
Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio;
oder R¹¹ und R¹² bilden gemeinsam eine C₃-C₁₂-Alkylenkette, welche ein bis drei C₁-C₄-Alkylgruppen tragen kann;
g) oder R¹ bildet einen Rest 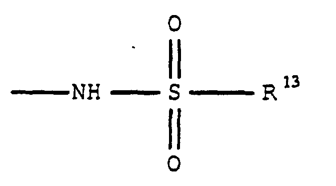 in dem R¹³ bedeutet:
C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio und/oder einen Phenylrest tragen können;
Phenyl, das durch ein bis fünf Halogenatome und/oder einen bis drei der folgenden Reste substituiert sein kann: Nitro, Cyano, C₁-C₄-Alkyl, C₁-C₄-Halogenalkyl, C₁-C₄-Alkoxy, C₁-C₄-Halogenalkoxy oder C₁-C₄-Alkylthio.

3. 3-Arylcarbonsäurederivate der Formel I gemäß Anspruch 1, in der R⁴ Phenyl, das wie in Anspruch 1 genannt substituiert sein kann, bedeutet und die restlichen Substituenten die in Anspruch 1 genannte Bedeutung haben.

4. 3-Arylcarbonsäurederivate der Formel I gemäß Anspruch 1, in der Z Sauerstoff, R⁴ Phenyl, das wie in Anspruch 1 genannt substituiert sein kann, R⁵ Methyl, X CH, R² und R³ Methoxy bedeuten und Y, R¹ und R⁶ die in Anspruch 1 genannte Bedeutung haben.

5. 3-Hetarylcarbonsäurederivate der. Formel I gemäß Anspruch 1, in der R⁴ ein fünf- oder sechsgliedriger Heteroaromat gemäß Anspruch 1 ist und die restlichen Substituenten die in Anspruch 1 genannte Bedeutung haben.

6. 3-Hetarylcarbonsäurederivate der Formel I gemäß Anspruch 1, in der Z Sauerstoff, R⁴ ein fünf- oder sechsgliedriger Heteroaromat gemäß Anspruch 1, R⁵ Methyl, X CH, R² und R³ Methoxy bedeuten und Y, R¹ und R⁶ die in Anspruch 1 genannte Bedeutung haben.

7. Herbizides Mittel, enthaltend eine Verbindung der Formel I gemäß Anspruch 1 und übliche inerte Zusatzstoffe.

8. Verfahren zur Bekämpfung unerwünschten Pflanzenwuchses, **dadurch gekennzeichnet, daß** man eine herbizid wirksame Menge einer Verbindung der Formel I gemäß Anspruch 1 auf die Pflanzen oder deren Lebensraum einwirken läßt.

9. Mittel zur Beeinflussung des Pflanzenwachstums, enthaltend eine Verbindung der Formel I gemäß Anspruch 1 und übliche inerte Zusatzstoffe.

10. Verfahren zur Regulierung des Pflanzenwachstums, **dadurch gekennzeichnet, daß** man eine bioregulatorisch wirksame Menge einer Verbindung der Formel I gemäß Anspruch 1 auf die Pflanzen oder deren Lebensraum einwirken läßt.

11. 3-(Het)arylcarbonsäurederivate der allgemeinen Formel VI, 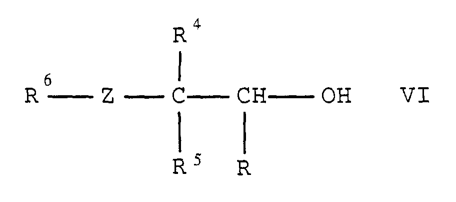 in der R, R⁴, R⁵, R⁶ und Z die in Anspruch 1 genannte Bedeutung haben mit der Maßgabe, daß R⁶ nicht unsubstituiertes C₁-C₈-Alkyl bedeutet, wenn R⁴ unsubstituiertes Phenyl oder 4-Isobutylphenyl und gleichzeitig R⁵ Methyl oder Wasserstoff bedeutet.

12. Verfahren zur Herstellung von 3-(Het)arylcarbonsäurederivaten der allgemeinen Formel VI, 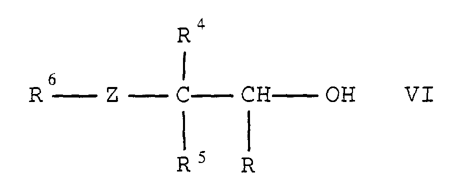 **dadurch gekennzeichnet, daß** man Epoxide der allgemeinen Formel IV, 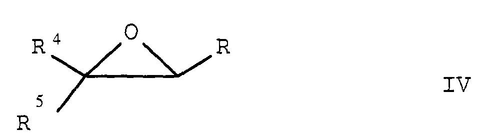 in der R, R⁴ und R⁵ die in Anspruch 1 genannte Bedeutung haben, mit Verbindungen der Formel V,
R⁶-ZH V
in der R⁶ und Z die in Anspruch 1 genannte Bedeutung haben, gegebenenfalls in einem inerten Lösungsmittel und/oder unter Zusatz eines geeigneten Katalysators, zur Reaktion bringt.

13. Verfahren zur Herstellung von 3-(Het)arylcarbonsäurederivaten der Formel I gemäß Anspruch 1, wobei Y Sauerstoff bedeutet, **dadurch gekennzeichnet, daß** man 3-Het(aryl)carbonsäurederivate der Formel VI, 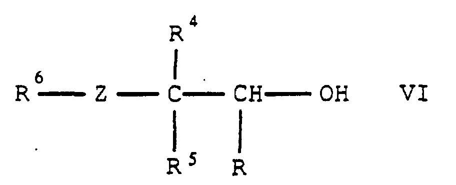 in der die Substituenten die in Anspruch 1 genannte Bedeutung haben, mit Pyrimidyl- oder Triazinylderivaten der allgemeinen Formel VII, 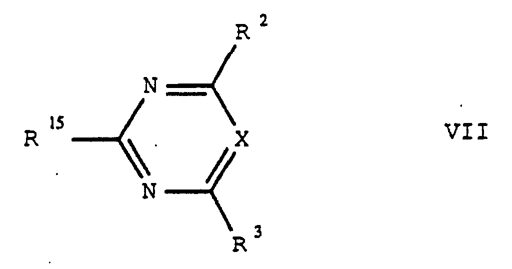 in der R¹⁵ Halogen oder R¹⁶SO₂- bedeutet, wobei R¹⁶ für C₁-C₄-Alkyl, C₁-C₄-Halogenalkyl oder Phenyl steht, in einem inerten Lösungsmittel in Gegenwart einer Base umsetzt.

14. Verfahren zur Herstellung von 3-Het(aryl)carbonsäurederivaten der Formel I gemäß Anspruch 1, wobei Y Schwefel bedeutet, **dadurch gekennzeichnet, daß** man 3-Het(aryl)carbonsäurederivate der allgemeinen Formel VIII, 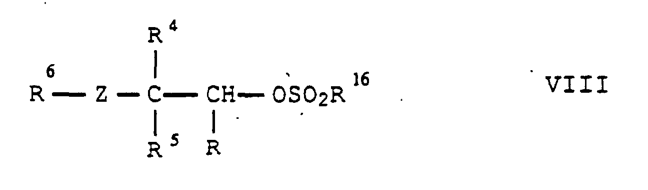 in der die Substituenten die in Anspruch 12 angegebene Bedeutung haben, mit Pyrimidyl- oder Triazinylthiolen der allgemeinen Formel IX, 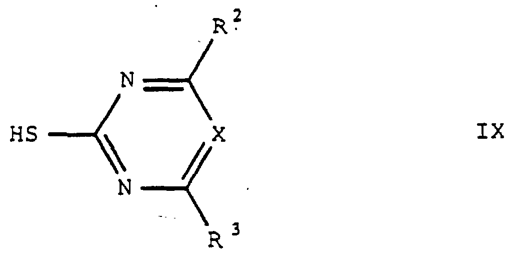 in der R², R³ und X die in Anspruch 1 genannte Bedeutung haben, zur Reaktion bringt.

## Claims

1. A 3-(het)arylcarboxylic acid derivative of the formula I 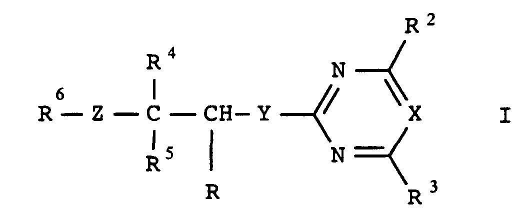 where
R is formyl, CO₂H or a radical hydrolyzable to COOH and
R² is C₁-C₄-alkyl, C₁-C₄-alkoxy;
X is CR¹⁴, where R¹⁴ is hydrogen;
R³ is C₁-C₄-alkyl, C₁-C₄-alkoxy;
R⁴ is phenyl which may be substituted by one or more, in particular one to three, of the following radicals: halogen, nitro, cyano, hydroxyl, mercapto, amino, C₁-C₄-alkyl, C₁-C₄-haloalkyl, C₁-C₄-alkoxy, C₁-C₄-haloalkoxy, C₁-C₄-alkylthio, C₁-C₄-alkylamino, di-C₁-C₄-alkylamino, C₁-C₄-alkylcarbonyl or C₁-C₄-alkoxycarbonyl;
a five-membered or six-membered heteroaromatic structure which contains one to three nitrogen atoms and/or one sulfur or oxygen atom and may carry one or more of the following radicals: halogen, nitro, cyano, hydroxyl, mercapto, amino, C₁-C₄-alkyl, C₁-C₄-haloalkyl, C₁-C₄-alkoxy, C₁-C₄-haloalkoxy, C₁-C₄-alkylthio, C₁-C₄-alkylamino, C₁-C₄-dialkylamino, C₁-C₄-alkylcarbonyl, C₁-C₄-alkoxycarbonyl or phenyl;
R⁵ is hydrogen, C₁-C₄-alkyl, C₃-C₈-cycloalkyl, C₁-C₄-haloalkyl or phenyl;
R⁶ is C₁-C₈-alkyl, it being possible for this radical to be mono- or polysubstituted by: phenyl or phenoxy which is mono- or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio;
Y is oxygen; and
Z is oxygen;
with the proviso that R⁶ is not unsubstituted C₁-C₄-alkyl when R⁴ is unsubstituted phenyl, Z is oxygen and simultaneously R⁵ is methyl or hydrogen.

2. A 3-(het)arylcarboxylic acid derivative of the general formula I as claimed in claim 1, where R is 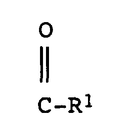 where R¹ has the following meanings:
a) hydrogen;
b) a succinylimidoxy group;
c) a 5-membered heteroaromatic structure which is bonded via a nitrogen atom, contains two or three nitrogen atoms and may carry one or two halogen atoms or one or two of the following radicals:
C₁-C₄-alkyl, C₁-C₄-haloalkyl, C₁-C₄-alkoxy,
C₁-C₄-haloalkoxy or C₁-C₄-alkylthio;
d) a radical 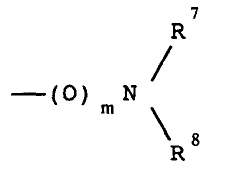 where m is 0 or 1 and R⁷ and R⁸, which may be identical or different, have the following meanings:
hydrogen;
C₁-C₈-alkyl, C₃-C₆-alkenyl, C₃-C₆-alkynyl or C₃-C₈-cycloalkyl, where each of these radicals may carry one to five halogen atoms or one or two of the following groups:
C₁-C₄-alkoxy, C₃-C₆-alkenyloxy, C₃-C₆-alkynyloxy,
C₁-C₄-alkylthio, C₃-C₆-alkenylthio, C₃-C₆-alkynylthio,
C₁-C₄-haloalkoxy, C₁-C₄-alkylcarbonyl,
C₃-C₆-alkenylcarbonyl, C₃-C₆-alkynylcarbonyl,
C₁-C₄-alkoxycarbonyl, C₃-C₆-alkenyloxycarbonyl,
C₃-C₆-alkynyloxycarbonyl,
di-C₁-C₄-alkylamino, C₃-C₈-cycloalkyl, phenyl or phenyl which is monosubstituted or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio;
phenyl which may be substituted by one or more of the following radicals: halogen, nitro, cyano, C₁-C₄-alkyl, C₁-C₄-haloalkyl, C₁-C₄-alkoxy, C₁-C₄-haloalkoxy or C₁-C₄-alkylthio;
R⁷ and R⁸ together form a cyclic, optionally substituted C₄-C₇-alkylene chain or together form a cyclic, optionally substituted C₃-C₆-alkylene chain containing a heteroatom selected from the group consisting of oxygen, sulfur and nitrogen;
e) R¹ is furthermore a group 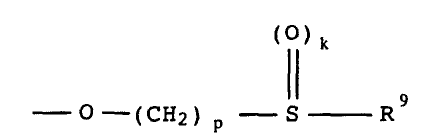 where R⁹ is C₁-C₄-alkyl, phenyl or phenyl which is monosubstituted or polysubstituted by halogen, nitro, cyano, C₁-C₄-alkyl, C₁-C₄-haloalkyl, C₁-C₄-alkoxy, C₁-C₄-haloalkoxy or C₁-C₄-alkylthio, or C₁-C₄-haloalkyl, C₃-C₆-alkenyl or C₃-C₆-alkynyl, p may be 1, 2, 3 or 4 and k may be 0, 1 or 2.
f) a radical OR¹⁰, where R¹⁰ is:
i) hydrogen, an alkali metal cation, one equivalent of an alkaline earth metal cation, the ammonium cation or an organic ammonium ion;
ii) C₃-C₈-cycloalkyl which may carry one to three C₁-C₄-alkyl radicals;
iii) C₁-C₈-alkyl which may carry one to five halogen atoms or one of the following radicals:
C₁-C₄-alkoxy, C₁-C₄-alkylthio, cyano,
C₁-C₄-alkylcarbonyl, C₃-C₈-cycloalkyl,
C₁-C₄-alkoxycarbonyl, phenyl, phenoxy or phenylcarbonyl, where the aromatic radicals in turn may each carry one to five halogen atoms or one to three of the following radicals: nitro, cyano,
C₁-C₄-alkyl, C₁-C₄-haloalkyl, C₁-C₄-alkoxy,
C₁-C₄-haloalkoxy or C₁-C₄-alkylthio;
iv) C₁-C₈-alkyl which may carry one to five halogen atoms and carries one of the following radicals: a 5-membered heteroaromatic structure containing one to three nitrogen atoms or a 5-membered heteroaromatic structure containing one nitrogen atom and one oxygen or sulfur atom, which may carry one to four halogen atoms or one or two of the following radicals: nitro, cyano, C₁-C₄-alkyl, C₁-C₄-haloalkyl, C₁-C₄-alkoxy, C₁-C₄-haloalkoxy or C₁-C₄-alkylthio;
v) C₂-C₆-alkyl which carries one of the following radicals in the 2 position: C₁-C₄-alkoxyimino, C₃-C₆-alkenyloxyimino, C₃-C₆-haloalkenyloxyimino or benzyloxyimino;
vi) C₃-C₆-alkenyl or C₃-C₆-alkynyl, where these groups in turn may carry one to five halogen atoms;
vii)phenyl which may carry one to five halogen atoms or one to three of the following radicals: nitro, cyano, C₁-C₄-alkyl, C₁-C₄-haloalkyl, C₁-C₄-alkoxy, C₁-C₄-haloalkoxy or C₁-C₄-alkylthio;
viii) a 5-membered heteroaromatic structure which has bonded via a nitrogen atom, contains one to three nitrogen atoms and may carry one or two halogen atoms or one or two of the following radicals: nitro, cyano, C₁-C₄-alkyl, C₁-C₄-haloalkyl, C₁-C₄-alkoxy, C₁-C₄-haloalkoxy or C₁-C₄-alkylthio;
ix) R¹⁰ is furthermore a group 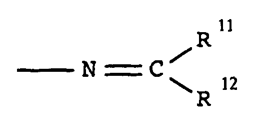 where R¹¹ and R¹², may be identical or different and are each:
C₁-C₈-alkyl, C₃-C₆-alkenyl, C₃-C₆-alkynyl or C₃-C₈-cycloalkyl, where these radicals may carry one C₁-C₄-alkoxy or C₁-C₄-alkylthio or one phenyl radical;
phenyl which may be substituted by one or more of the following radicals:
halogen, nitro, cyano, C₁-C₄-alkyl, C₁-C₄-haloalkyl, C₁-C₄-alkoxy, C₁-C₄-haloalkoxy or C₁-C₄-alkylthio;
or R¹¹ and R¹² together form a C₃-C₁₂-alkylene chain which may carry one to three C₁-C₄-alkyl groups;
g) or R¹ forms a radical 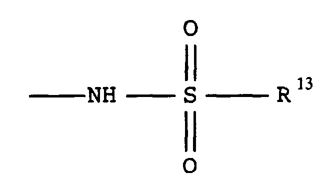 where R¹³ is:
C₁-C₄-alkyl, C₃-C₆-alkenyl, C₃-C₆-alkynyl or C₃-C₈-cycloalkyl, where these radicals may carry one C₁-C₄-alkoxy or C₁-C₄-alkylthio or one phenyl radical;
phenyl which may be substituted by one to five halogen atoms or one to three of the following radicals: nitro, cyano, C₁-C₄-alkyl, C₁-C₄-haloalkyl, C₁-C₄-alkoxy, C₁-C₄-haloalkoxy or C₁-C₄-alkylthio.

3. A 3-arylcarboxylic acid derivative of the formula I as claimed in claim 1, in which R⁴ is phenyl which may be substituted as stated in claim 1 and the remaining substituents have the meanings stated in claim 1.

4. A 3-arylcarboxylic acid derivative of the formula I as claimed in claim 1, in which Z is oxygen, R⁴ is phenyl which may be substituted as stated in claim 1, R⁵ is methyl, X is CH, R² and R³ are each methoxy and Y, R¹ and R⁶ have the meanings stated in claim 1.

5. A 3-hetarylcarboxylic acid derivative of the formula I as claimed in claim 1, in which R⁴ is a five- or six-membered heteroaromatic structure as claimed in claim 1 and the remaining substituents have the meanings stated in claim 1.

6. A 3-hetarylcarboxylic acid derivative of the formula I as claimed in claim 1, in which Z is oxygen, R⁴ is a five- or six-membered heteroaromatic structure as claimed in claim 1, R⁵ is methyl, X is CH, R² and R³ are methoxy and Y, R¹ and R⁶ have the meanings stated in claim 1.

7. A herbicide containing a compound of the formula I as claimed in claim 1 and conventional inert additives.

8. A method for controlling undesirable plant growth, wherein a herbicidal amount of a compound of the formula I as claimed in claim 1 is allowed to act on the plants or on their habitat.

9. An agent for influencing plant growth, containing a compound of the formula I as claimed in claim 1 and conventional inert additives.

10. A method for regulating plant growth, wherein a bioregulatory amount of a compound of the formula I as claimed in claim 1 is allowed to act on the plants or on their habitat.

11. A 3-(het)arylcarboxylic acid derivative of the formula VI 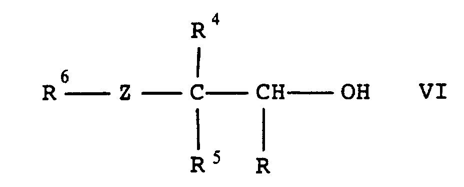 where R, R⁴, R⁵, R⁶ and Z have the meanings stated in claim 1, with the proviso that R⁶ is not unsubstituted C₁-C₈-alkyl where R⁴ is unsubstituted phenyl or 4-isobutylphenyl and simultaneously R⁵ is methyl or hydrogen.

12. A process for the preparation of a 3-(het)arylcarboxylic acid derivative of the formula VI 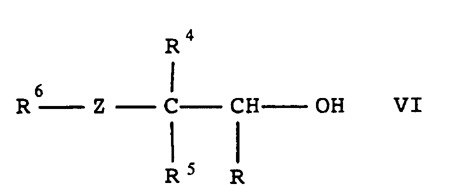 wherein an epoxide of the formula IV 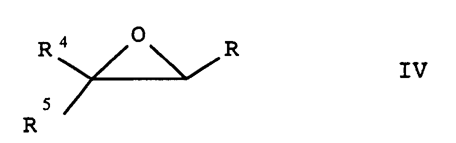 where R, R⁴ and R⁵ have the meanings stated in claim 1, is reacted with a compound of the formula V
R⁶-ZH V
where R⁶ and Z have the meanings stated in claim 1, if required in an inert solvent or with the addition of a suitable catalyst.

13. A process for the preparation of 3-(het)arylcarboxylic acid derivatives of the formula I as claimed in claim 1, where Y is oxygen wherein the 3-het(aryl)carboxylic acid derivative of the formula VI 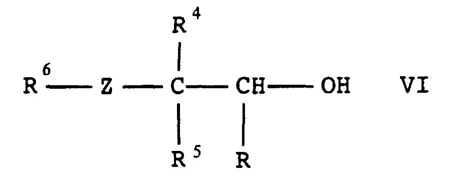 where the substituents have the meanings stated in claim 1, is reacted with a pyrimidyl or triazinyl derivative of the formula VII 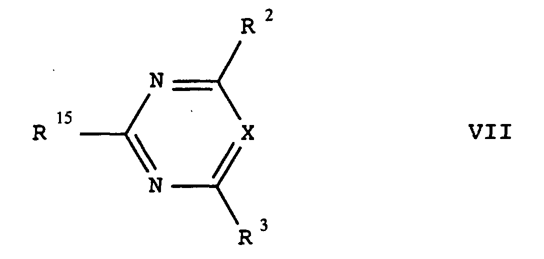 where R¹⁵ is halogen or R¹⁶SO₂- and R¹⁶ is C₁-C₄-alkyl, C₁-C₄-haloalkyl or phenyl, in an inert solvent in the presence of a base.

14. A process for the preparation of a 3-het(aryl)carboxylic acid derivative of the formula I as claimed in claim 1, where Y is sulfur, wherein a 3-het(aryl)carboxylic acid derivative of the formula VIII 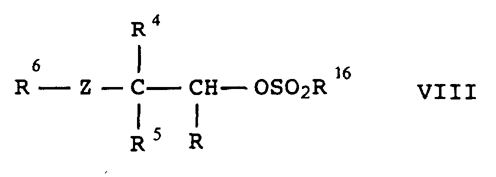 where the substituents have the meanings stated in claim 12, is reacted with a pyrimidyl- or triazinylthiol of the formula IX 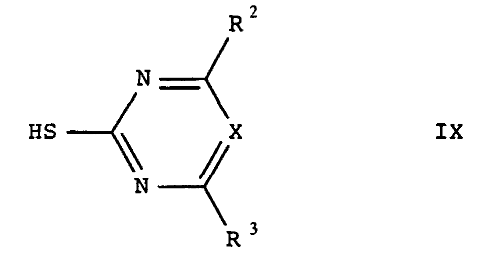 where R², R³ and X have the meanings stated in claim 1.

## Revendications

1. Dérivés d'acides 3-(hétéro)aryl-carboxyliques de formule générale I 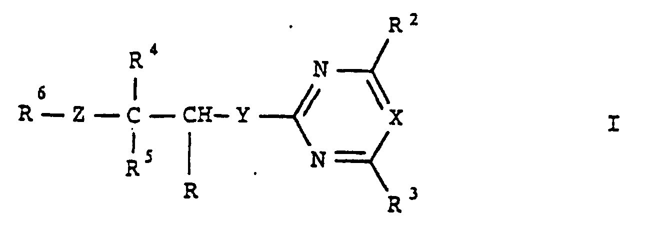 dans laquelle R représente un groupe formyle, un groupe CO₂H ou un groupe hydrolysable en groupe COOH et les autres symboles ont les significations suivantes :
R² : un groupe alkyle en C1-C4, alcoxy en C1-C4 ;
X : un groupe CR¹⁴ dans lequel R¹⁴ représente l'hydrogène,
R³ : un groupe alkyle en C1-C4, alcoxy en C1-C4,
R⁴ : un groupe phényle qui peut porter un ou plusieurs, plus spécialement un à trois des substituants suivants : halogéno, nitro, cyano, hydroxy, mercapto, amino, alkyle en C1-C4, halogénoalkyle en C1-C4, alcoxy en C1-C4, halogénoalcoxy en C1-C4, alkylthio en C1-C4, alkylamino en C1-C4, di-(alkyle en C1-C4)amino, (alkyle en C1-C4)carbonyle ou (alcoxy en C1-C4)cabonyle ;
un cycle hétéroaromatique à cinq ou six chaînons contenant un à trois atomes d'azote et/ou un atome de soufre ou d'oxygène et qui peut porter un ou plusieurs des substituants suivants : halogéno, nitro, cyano, hydroxy, mercapto, amino, alkyle en C1-C4, halogénoalkyle en C1-C4, alcoxy en C1-C4, halogénoalcoxy en C1-C4, alkylthio en C1-C4, alkylamino en C1-C4, di-(alkyle en C1-C4)amino, (alkyle en C1-C4)carbonyle, (alcoxy en C1-C4)carbonyle ou phényle ;
R⁵ : l'hydrogène, un groupe alkyle en C1-C4, cycloalkyle en C3-C8, halogénoalkyle en C1-C4 ou phényle ;
R⁶ : un groupe alkyle en C1-C8 qui peut porter un ou plusieurs substituants phényle, phényle ou phénoxy portant lui-même un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4 ;
Y : l'oxygène ;
Z : l'oxygène ;
sous réserve que R⁶ ne peut représenter un groupe alkyle en C1-C4 non substitué lorsque R⁴ représente un groupe phényle non substitué, Z l'oxygène et, simultanément, R⁵ un groupe méthyle ou l'hydrogène.

2. Dérivés d'acides 3-(hétéo)aryl-carboxyliques de formule générale I de la revendication 1 dans laquelle R représente un groupe 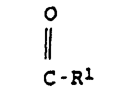 dans lequel R¹ a l'une des significations suivantes :
a) l'hydrogène ;
b) un groupe succinylimidoxy ;
c) un cycle hétéroaromatique à cinq chaînons relié par l'intermédiaire d'un atome d'azote, qui contient deux à trois atomes d'azote et peut porter un à deux atomes d'halogènes et/ou un à deux des substituants suivants :
alkyle en C1-C4, halogénoalkyle en C1-C4, alcoxy en C1-C4, halogénoalcoxy en C1-C4 et/ou alkylthio en C1-C4 ;
d) un groupe 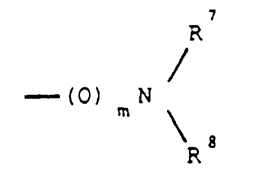 dans lequel m est égal à 0 ou 1 et R⁷ et R⁸, ayant des significations identiques ou différentes, représentent chacun :
l'hydrogène ;
un groupe alkyle en C1-C8, alcényle en C3-C6, alcynyle en C3-C6, cycloalkyle en C3-C8, ces groupes pouvant porter chacun un à cinq atomes d'halogènes et/ou un à deux des substituants suivants : alcoxy en C1-C4, alcényloxy en C3-C6, alcynyloxy en C3-C6, alkylthio en C1-C4, alcénylthio en C3-C6, alcynylthio en C3-C6, halogénoalcoxy en C1-C4, (alkyle en C1-C4)carbonyle, (alcényle en C3-C6)carbonyle, (alcynyle en C3-C6)carbonyle, (alcoxy en C1-C4)carbonyle, (alcényle en C3-C6)oxycarbonyle, (alcynyle en C3-C6)oxycarbonyle, di-(alkyle en C1-C4)amino, cycloalkyle en C3-C8, phényle, phényle portant lui-même un ou deux substituants halogéno, nitro, cyano, alkyle en C1-C4, halogénoalkyle en C1-C4, alcoxy en C1-C4, halogénoalcoxy en C1-C4 ou alkylthio en C1-C4 ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4,
R⁷ et R⁸ peuvent former ensemble une chaîne alkylène en C4-C7 fermée en un cycle et éventuellement substituée ou bien une chaîne alkylène en C3-C6 fermée en un cycle et éventuellement substituée qui contient un hétéroatome choisi parmi l'oxygène, le soufre et l'azote ;
e) R¹ peut en outre représenter un groupe 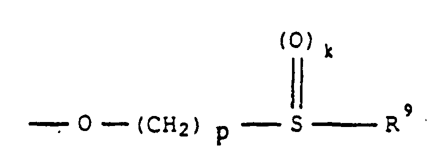 dans lequel R⁹ représente un groupe alkyle en C1-C4, un groupe phényle, un groupe phényle portant un ou plusieurs substituants halogéno, nitro, cyano, alkyle en C1-C4, halogénoalkyle en C1-C4, alcoxy en C1-C4, halogénoalcoxy en C1-C4 ou alkylthio en C1-C4, un groupe halogénoalkyle en C1-C4, alcényle en C3-C6 ou alcynyle en C3-C6, p est égal à 1, 2, 3 ou 4 et k à 0, 1 ou 2 ;
f) un groupe OR¹⁰ dans lequel R¹⁰ représente :
i) l'hydrogène, un cation de métal alcalin, l'équivalent d'un cation de métal alcalino-terreux, le cation ammonium ou un ion ammonium organique ;
ii) un groupe cycloalkyle en C3-C8 qui peut porter un à trois substituants alkyle en C1-C4 ;
iii) un groupe alkyle en C1-C8 qui peut porter un à cinq atomes d'halogènes et/ou un des substituants suivants :
alcoxy en C1-C4, alkylthio en C1-C4, cyano, (alkyle en C1-C4)carbonyle, cycloalkyle en C3-C8, (alcoxy en C1-C4)carbonyle, phényle, phénoxy ou phénylcarbonyle, les radicaux aromatiques pouvant eux-mêmes porter un à cinq atomes d'halogènes et/ou un à trois des substituants suivants : nitro, cyano, alkyle en C1-C4, halogénoalkyle en C1-C4, alcoxy en C1-C4, halogénoalcoxy en C1-C4 et/ou alkyklthio en C1-C4 :
iv) un groupe alkyle en C1-C8 qui peut porter un à cinq atomes d'halogènes et un des substituants suivants : un cycle hétéroaromatique à cinq chaînons contenant un à trois atomes d'azote ou un cycle hétéroaromatique à cinq chaînons contenant un atome d'azote et un atome d'oxygène ou de soufre, qui peut porter un à quatre atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
v) un groupe alkyle en C2-C6 portant en position 2 l'un des substituants suivants : alcoxyimino en C1-C4, alcényloxyimino en C3-C6, halogénoalcényloxyimino en C3-C6 ou benzyloxyimino ;
vi) un groupe alcényle en C3-C6 ou un groupe alcynyle en C3-C6, ces groupes pouvant porter eux-mêmes un à cinq atomes d'halogènes ;
vii) un groupe phényle qui peut porter un à cinq atomes d'halogènes et/ou un à trois des substituants suivants : nitro, cyano, alkyle en C1-C4, halogénoalkyle en C1-C4, alcoxy en C1-C4, halogénoalcoxy en C1-C4 et/ou alkylthio en C1-C4 ;
viii) un cycle hétéroaromatique à cinq chaînons relié par l'intermédiaire d'un atome d'azote, qui contient un à trois atomes d'azote et peut porter un à deux atomes d'halogènes et/ou un à deux des substituants suivants : nitro, cyano, alkyle en C1-C4, halogénoalkyle en C1-C4, alcoxy en C1-C4, halogénoalcoxy en C1-C4 et/ou alkylthio en C1-C4 ;
ix) R¹⁰ peut en outre représenter un groupe 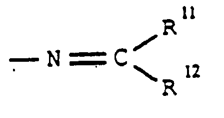 dans lequel R¹¹ et R¹², ayant des significations identiques ou différentes, représentent chacun :
un groupe alkyle en C1-C8, alcényle en C3-C6, alcynyle en C3-C6, cycloalkyle en C3-C8, ces groupes pouvant porter un substituant alcoxy en C1-C4, alkylthio en C1-C4 et/ou phényle ;
un groupe phényle qui peut porter un ou plusieurs des substituants suivants : halogéno, nitro, cyano, alkyle en C1-C4, halogénoalkyle en C1-C4, alcoxy en C1-C4, halogénoalcoxy en C1-C4 ou alkylthio en C1-C4 ;
ou bien R¹¹ et R¹² forment ensemble une chaîne alkylène en C3-C12 qui peut porter un à trois groupes alkyle en C1-C4 ;
g) ou bien R¹ représente un groupe 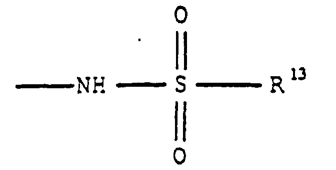 dans lequel R¹³ représente :
un groupe alkyle en C1-C4, alcényle en C3-C6, alcynyle en C3-C6, cycloalkyle en C3-C8, ces groupes pouvant porter un substituant alcoxy en C1-C4, alkylthio en C1-C4 et/ou phényle ;
un groupe phényle qui peut porter un à cinq atomes d'halogènes et/ou un à trois des substituants suivants : nitro, cyano, alkyle en C1-C4, halogénoalkyle en C1-C4, alcoxy en C1-C4, halogénoalcoxy en C1-C4 ou alkylthio en C1-C4.

3. Dérivés d'acides 3-arylcarboxyliques de formule I de la revendication 1 dans laquelle R⁴ représente un groupe phényle qui peut porter les substituants mentionnés dans la revendication 1 et les autres symboles ont les significationsk indiquées dans la revendication 1.

4. Dérivés d'acides 3-arylcarboxyliques de formule I de la revendication 1 dans laquelle Z représente l'oxygène, R⁴ un groupe phényle qui peut porter les substituants mentionnés dans 1a revendication 1, R⁵ un groupe méthyle, X un groupe CH, R² et R³ des groupes méthoxy, et Y, R¹ et R⁶ ont les significations indiquées dans la revendication 1.

5. Dérivés d'acides 3-hétéroarylcarboxyliques de formule I de la revendication 1 dans laquelle R⁴ représente un cycle hétéroaromatique à cinq ou six chaînons selon la revendication 1 et les autres symboles ont les significations indiquées dans la revendication 1.

6. Dérivés d'acides 3-hétéroarylcarboxyliques de formule I de la revendication 1 dans laquelle Z représente l'oxygène, R4 un cycle hétéroaromatique à cinq ou six chaînons selon la revendication 1, R⁵ un groupe méthyle, X un groupe CH, R² et R³ des groupes méthoxy et Y, R¹ et R⁶ ont les significations indiquées dans la revendication 1.

7. Produit herbicide contenant un composé de formule I de la revendication 1 et des additifs inertes usuels.

8. Procédé pour combattre les croissances des végétaux indésirables, **caractérisé par le fait que** l'on fait agir une quantité herbicide efficace d'un composé de formule I de la revendication 1 sur les végétaux ou leur habitat.

9. Produit pour agir sur la croissance de végétaux, contenant un composé de formule I de la revendication 1 et des additifs inertes usuels.

10. Procédé pour la régulation de la croissance des végétaux, **caractérisé par le fait que** l'on fait agir une quantité biorégulatrice efficace d'un composé de formule I de la revendication 1 sur les végétaux ou leur habitat.

11. Dérivés d'acides 3-(hétéro)arylcarboxyliques de formule générale VI 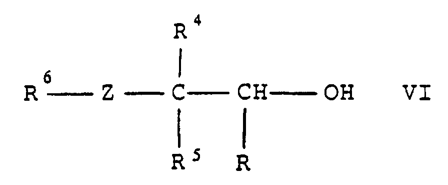 dans laquelle R, R⁴, R⁵, R⁶ et Z ont les significations indiquées dans la revendication 1 sous réserve que R⁶ ne peut représenter un groupe alkyle en C1-C8 non substitué lorsque R⁴ représente un groupe phényle non substitué ou un groupe 4-isobutylphényle et R⁵, simultanément, un groupe méthyle ou l'hydrogène.

12. Procédé pour la préparation des dérivés d'acides 3-(hétéro)arylcarboxyliques de formule générale VI 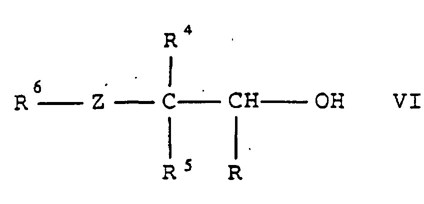 **caractérisé par le fait que** l'on fait réagir des époxydes de formule générale IV 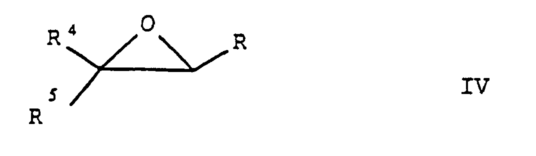 dans laquelle R, R⁴ et R⁵ ont les significations indiquées dans la revendication 1, avec des composés de formule V
R⁶-ZH V
dans laquelle R⁶ et Z ont les significations indiquées dans la revendication 1, éventuellement dans un solvant inerte et/ou avec adjonction d'un catalyseur approprié.

13. Procédé pour la préparation des dérivés d'acides 3-(hétéro)arylcarboxyliques de formule I de la revendication I, dans laquelle Y représente l'oxygène, **caractérisé par le fait que** l'on fait réagir des dérivés d'acides 3-hétéro(aryl)carboxyliques de formule VI 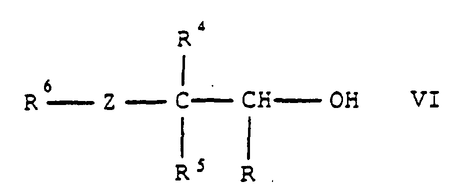 dans laquelle les symboles ont les significations indiquées dans la revendication 1, avec des dérivés pyrimidyliques ou triazinyliques de formule générale VII 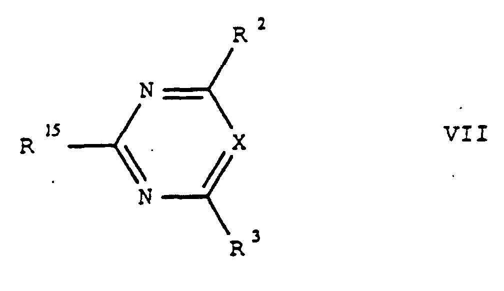 dans laquelle R¹⁵ représente un halogène ou un groupe R¹⁶SO₂- dans lequel R¹⁶ représente un groupe alkyle en C1-C4, halogénoalkyle en C1-C4 ou phényle, dans un solvant inerte et en présence d'une base.

14. Procédé pour la préparation des dérivés d'acides 3-hétéro(aryl)carboxyliques de formule I de la revendication 1, dans laquelle Y représente le soufre, **caractérisé par le fait que** l'on fait réagir des dérivés d'acides 3-hétéro(aryl)carboxyliques de formule générale VIII 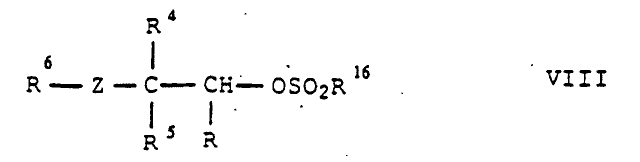 dans laquelle les symboles ont les significations indiquées dans la revendication 12, avec des pyrimidyl- ou triazinyl-thiols de formule générale IX 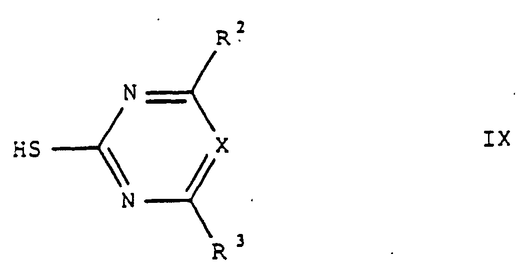 dans laquelle R², R³ et X ont les significations indiquées dans la revendication 1.
